# EUROPEAN PATENT APPLICATION

(11) **EP 2 689 687 A1**
(43) Date of publication of application: **29.01.2014**
(21) Application number: 12760730.7
(22) Date of filing: 15.02.2012
(51) Int. Cl.: A45D 26/00, A61N 5/06

(54) **LIGHT-IRRADIATING BEAUTY DEVICE**

(30) Priority: 23.03.2011 JP 2011063661
(71) Applicant: Panasonic Corporation, Osaka 571-8501 (JP)
(72) Inventor: SUEYOSHI, Hidekazu, Osaka 540-6207 (JP); INOUE, Tomoyuki, Osaka 540-6207 (JP); MURAKI, Kenichi, Osaka 540-6207 (JP)
(74) Representative: Grünecker, Kinkeldey, Stockmair & Schwanhäusser
(86) International application number: PCT/JP2012/053503
(87) International publication number: WO 2012/127941

(57) **Abstract**

A light-irradiating beauty device includes: a cutter; and a light-irradiating part that is placed beside the cutter, and is configured to irradiate light. The cutter includes: a retractable trimmer blade including a movable blade and a stationary blade; an operation means configured to protrude the trimmer blade from the beauty device or retract the trimmer blade into the beauty device; and a driver for driving the movable blade along the stationery blade. The driver stops moving the movable blade when the trimmer blade has been retracted into the beauty device by the operation means.

## Description

### Technical Field

The invention relates to a light- irradiating beauty device.

### Background Art

Conventionally, light-irradiating beauty devices are known that include an epilation part and a light-irradiating part, which irradiates light on a body surface (see for example JP 2002-360336A, hereinafter referred to as Document 1).

The device described in Document 1 includes an epilating actuator, and a semiconductor laser diode, which is provided alongside the epilating actuator and irradiates light on a body The device irradiates the laser beam on the skin with the semiconductor laser diode while epilating the hair grown on the body surface with the epilating actuator.

In the device described in Document 1, the epilating actuator is fixed in place, and is configured to always operate along the semiconductor laser diode.

In the device described in Document 1, the epilating actuator and the semiconductor laser diode always operate at the same time. Therefore, there is a problem in that the epilating actuator is pressed on not only the body surface with hair but also the body surface without hair.

### Summary of Invention

The present invention has been made in the light of the above-described circumstances, and the object of the present invention is to improve the usability of a light-irradiating part when a cutter is not used, in a device having the cutter and the light-irradiating part.

The present invention relates to a light-irradiating beauty device that includes: a cutter (3); and a light-irradiating part (4) that is placed beside the cutter (3), and is configured to irradiate light. The cutter (3) includes: a retractable trimmer blade (30) including a movable blade (301) and a stationary blade (300); an operation means (31) configured to protrude the trimmer blade (30) from the light-irradiating beauty device or retract the trimmer blade (30) into the light-irradiating beauty device; and a driver (32) configured to drive the movable blade (301) along the stationary blade (300). The driver (32) is configured to stop moving the movable blade (301) when the trimmer blade (30) has been retracted into the light-irradiating beauty device by the operation means (31).

In one embodiment, the light-irradiating part (4) is configured to irradiate light regardless of a position of the trimmer blade (30) relative to the light-irradiating beauty device.

In one embodiment, the light-irradiating device further includes a frame (12) to which the cutter (3) is attached. The operation means (31) includes: a connection portion connected to the trimmer blade (30) via a through-hole (21) of the flame (12); and a slidable lever (310) provided on the flame (12). The trimmer blade (30) is configured to protrude from or retract into the light-irradiating beauty device depending on a slide of the lever (310).

In one embodunent, the light-irradiating part (4) includes: an emission portion (44); and a contact portion (40) is free to move in a direction of a light axis (44a) extending from the emission portion (44). An end face of the contact portion (40) on a base end side of the light axis (44a) is substantially flush with a tip end of the trimmer blade (30) protruded from the light-irradiating beauty device.

In the light-irradiating beauty device according to the present invention, it is possible to improve the usability of the light-irradiating part when the cutter is not used, in the device having the cutter and the light-irradiating part.

### Brief Description of Drawings

Preferable embodiments according to the present invention will be described in detail. Other features and advantages of the present invention will become apparent from the following detailed description with reference to the attached drawings:
FIG. 1 is a side sectional view of the present embodiment, illustrating a state in which a cutter is protruded from a beauty device main body;
FIG. 2 is a side sectional view in which a part is omitted, illustrating a state in which the cutter is retracted into the beauty device main body;
FIG. 3A is a plan view of the light-irradiating beauty device according to the present embodiment, and FIG. 3B is a side view of the beauty device main body;
FIG. 4A is a front view of the beauty device main body according to the present embodiment, and FIG. 4B is a side view of the beauty device main body;
FIG. 5 is an exploded perspective view of a frame, a light-irradiating pant, and the cutter according to the present embodiment;
FIG. 6 is an exploded perspective view of the frame according to the present embodiment viewed from a back face side;
FIG. 7 is a cross-section view taken along line A-A in FIG. 4B;
FIG. 8 is a side sectional view of a main portion illustrating details of the cutter according to the present embodiment;
FIG. 9 is an exploded perspective view illustrating the vicinity of a driver and a cooling unit according to the present embodiment;
FIG. 10 is a cross section view taken along line B-B in FIG. 4B;
FIG. 11 is an exploded perspective view of the beauty device main body according to the present embodiment;
FIG. 12 is an exploded perspective view of a light irradiating unit according to the present embodiment; and
FIG. 13 is a side view of the light-irradiating beauty device in use according to the present embodiment.

### Description of Embodiments

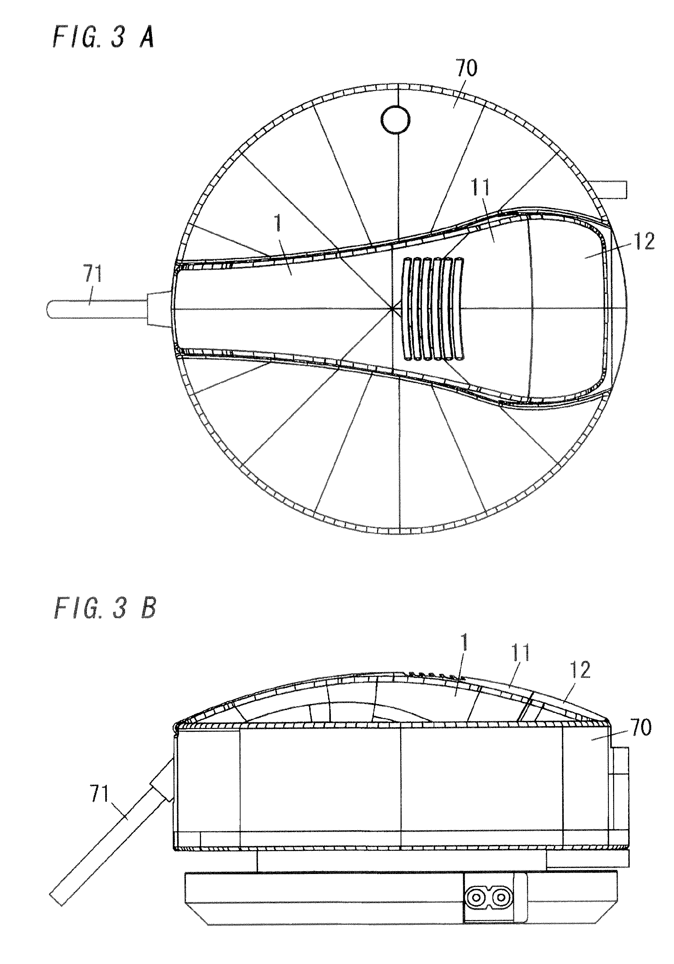

After epilating hair grown on skin of a body of a human or an animal, or the like (hereinafter referred to as "body surface 9"), a light-irradiating beauty device according to the present embodiment suppresses growth or regeneration of the hair on the body surface 9 (hereinafter referred to as "hair-growth-regeneration suppression") by irradiating light thereon. As shown in FIG. 3, the light-irradiating beauty device according to the present embodiment includes a beauty device main body 1 that performs hair-growth-regeneration suppression treatment on the body surface 9, and a power supply unit 70 that is connected to the beauty device main body 1 through a cable 71. In the light-irradiating beauty device according to the present embodiment, a charge storage unit, which may be constituted by a capacitor or the like, and a controller are built into the power supply unit 70, and the beauty device main body 1 is formed compactly.

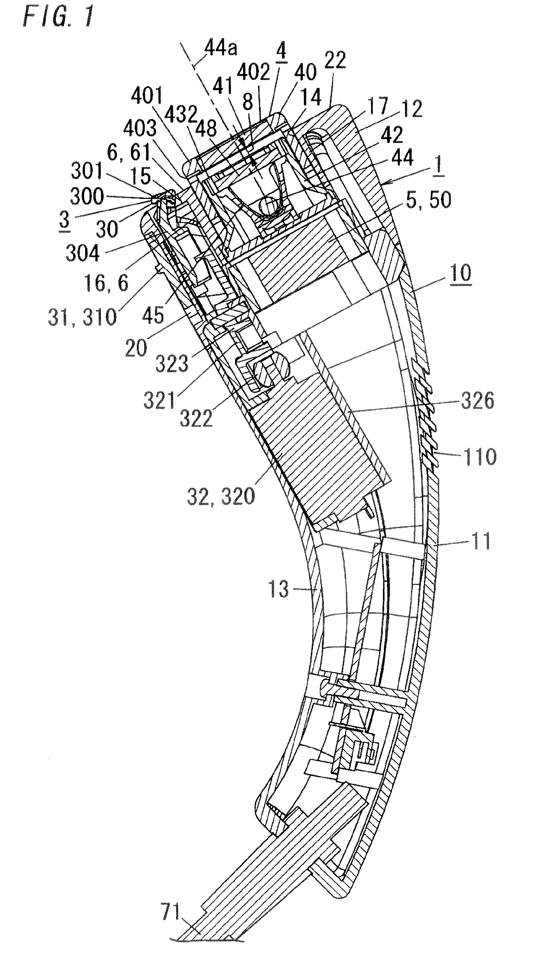

As shown in FIG. 1, the beauty device main body 1 includes a casing 10 that forms its outer shell, a cutter 3 that is accommodated in the casing 10 and cuts hair grown on the body surface 9, and a light-irradiating part 4 that is accommodated in the casing 10 and is configured to irradiate light on the body surface 9. That is to say, the light-irradiating part 4 includes an emission member 44. The beauty device main body 1 further includes a cooling unit 5 for cooling the light-irradiating part 4.

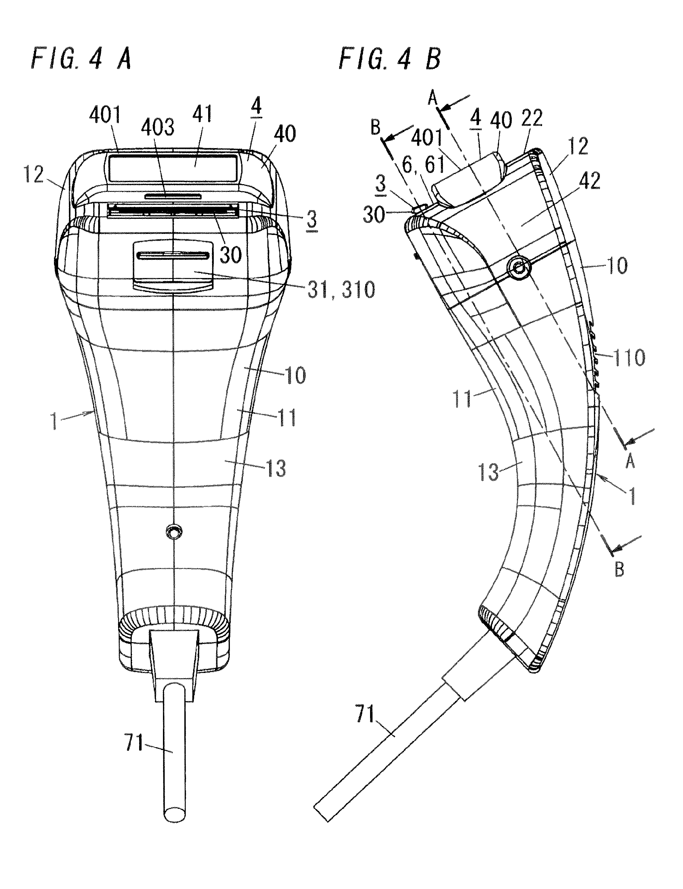

As shown in FIG. 4, the casing 10 is configured in a shape that becomes thinner towards its lower end. The casing 10 is formed in an arc-like shape that curves sideward. Specifically, the cutter 3 is located radially inward, and the light-irradiating part 4 is located radially outward. The casing 10 is constituted by a main body 11 that constitutes a lower portion and a center portion of the casing 10 and has a gripping portion 13, and a frame 12 that constitutes an upper portion of the casing 10. The gripping portion 13 of the main body 11 is configured to such an outer diameter that a user can hold it in one hand. The frame 12 is provided detachably with respect to the main body 11.

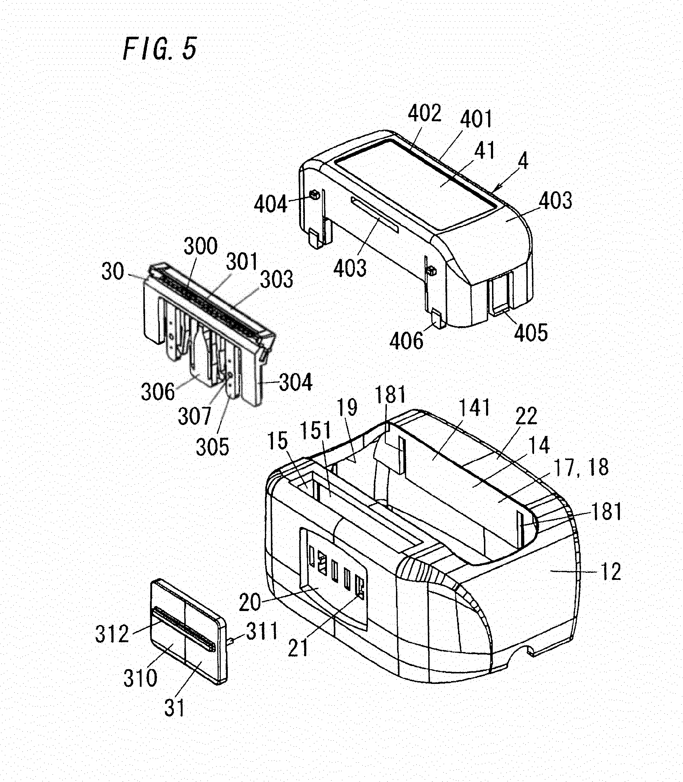

The frame 12 is formed in the shape of a box with a lid that is open to the lower side. As shown in FIG. 5, the frame 12 includes a first mounting part 14 to which the light-irradiating part 4 is attached, and a (second) mounting part 15 to which the cutter 3 is attached.

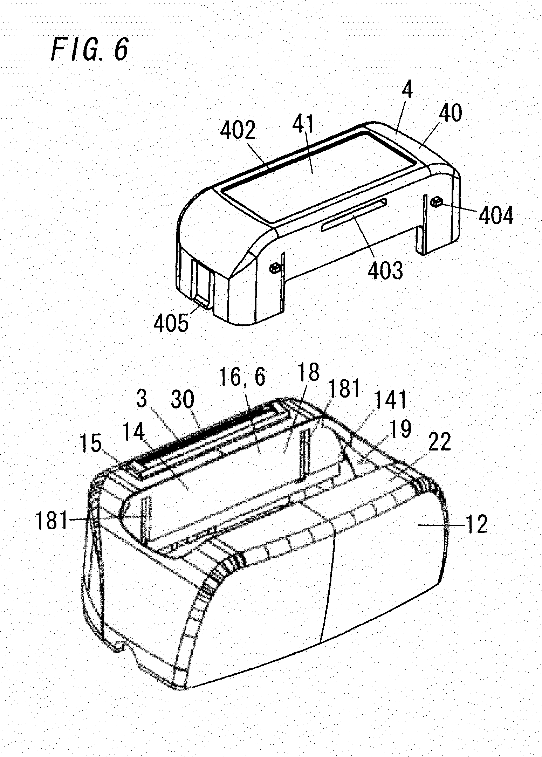

The mounting part 14 is a portion for mounting the light-irradiating part 4, and specifically holds a light-shielding tube portion (contact portion) 40 movable along the light axis 44a extending from the emission member 44 (hereinafter referred to as "light axis direction"). The mounting part 14 has an attachment opening 141 of elongated rectangular shape in a plan view that runs through the first mounting part 14 in the light axis direction (vertical direction). As shown in FIGS. 5 and 6, the mounting part 14 includes a partition wall 16 located between the mounting part 14 and the mounting part 15, and a vertical wall 17 that is located opposite to the partition wall 16. The partition wall 16 and the vertical wall 17 constitute a pair of guide faces 18 that guide a movement of the light-shielding tube portion 40 in the light axis direction. The guide faces 18 include a plurality of guide grooves 181 that respectively guide a movement of a plurality of guide protrusions 404 provided at the light-shielding tube portion 40. The guide grooves 181 are formed lengthwise parallel to the light axis direction. Moreover, the mounting part 14 has, at an inner circumferential edge of the attachment opening 141, a counter-engaging portion 19 that engages an engaging portion 405 provided at the light-shielding tube portion 40. The counter-engaging portion 19 keeps the light-shielding tube portion 40 from protruding beyond a predetermined position.

As shown in FIG. 6, the partition wall 16 is provided to protrude downward from a rear face of an end face portion 22 of the frame 12. The partition wall 16 is provided so that its edge is flush with the inner circumferential edge of the attachment opening 141 of the mounting part 14. The partition wall 16 is located between the light-irradiating part 4 and the cutter 3, and prevents hair scraps cut by the cutter 3 from entering the light-irradiating part 4.

The mounting part 15 is a portion to accommodate a trimmer blade 30, which constitutes the cutter 3, movable parallel to the light axis direction. As shown in FIG. 5, the mounting part 15 includes an attachment opening 151 that extends parallel to the light axis direction. The mounting part 15 is located adjacent to the mounting part 14. The attachment opening 151 of the second mounting part 15 has an elongated slit shape, and the longitudinal direction thereof is approximately parallel to the longitudinal direction of the attachment opening 141 of the mounting part 14.

The frame 12 has a lever mounting portion 20 at a side face thereof, to which a lever 310 (details will be described later) of the cutter 3 is mounted. The lever mounting portion 20 has a plurality of (for example two) through-holes 21 that pierce therethrough for inserting respectively a plurality of (for example two) connection portions 311 of the lever 310. The through-holes 21 are configured by long holes elongated parallel to the light axis direction. The lever 310 is attached to the lever mounting portion 20 such that it is slidable parallel to the light axis direction. Note that the lever 310 of the cutter 3 will be described later in detail.

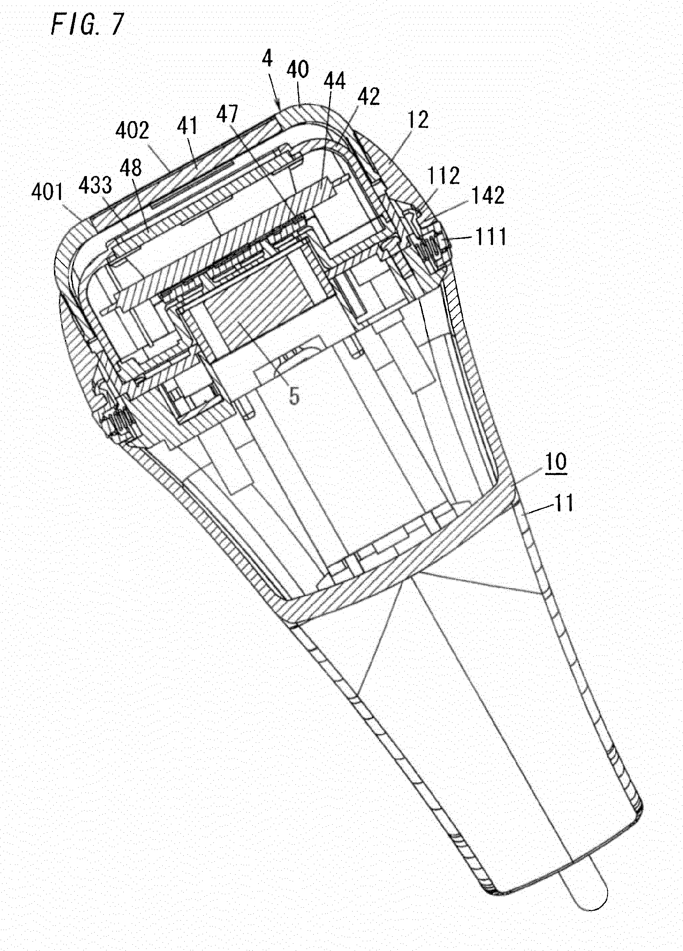

The frame 12 is detachably engaged with the main body 11. As shown in FIG. 7, the frame 12 has a pair of locking portions 142 provided respectively at opposing side faces. The locking portions 142 are configured to engage respective counter-locking portions 112 provided at the main body 11. The counter-locking portions 112 of the main body 11 are respectively provided at release portions 111, which are elastically biased outward, and when a user presses the release portions 111 inward, the engaging state with the locking portions 142 is released.

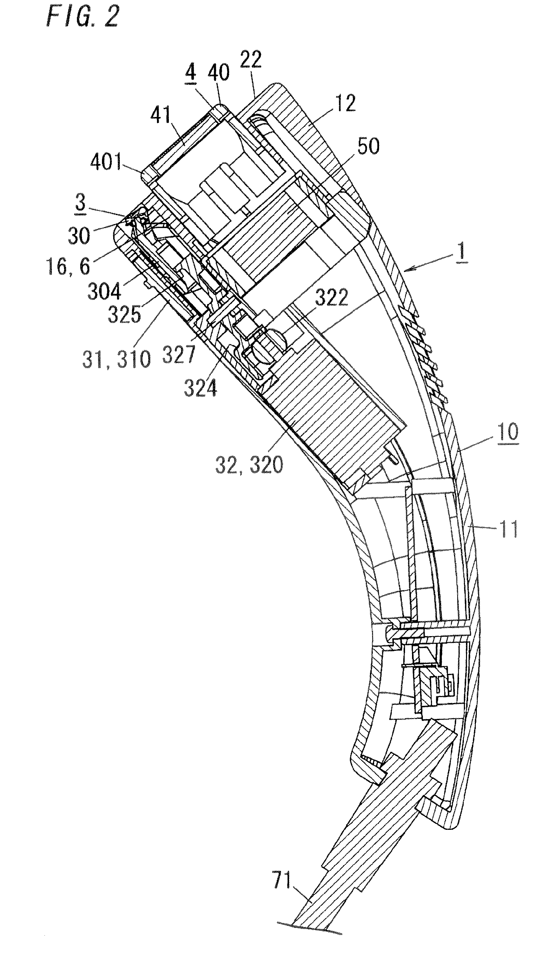

The cutter 3 is used to cut hair grown on a body surface 9. As shown in FIGS. 1 and 2, the cutter 3 according to the present embodiment includes a trimmer blade 30 that can be retracted in parallel to the light axis direction, an operation means 31 for moving the trimmer blade 30, and a driver 32 that is configured to drive the trimmer blade 30.

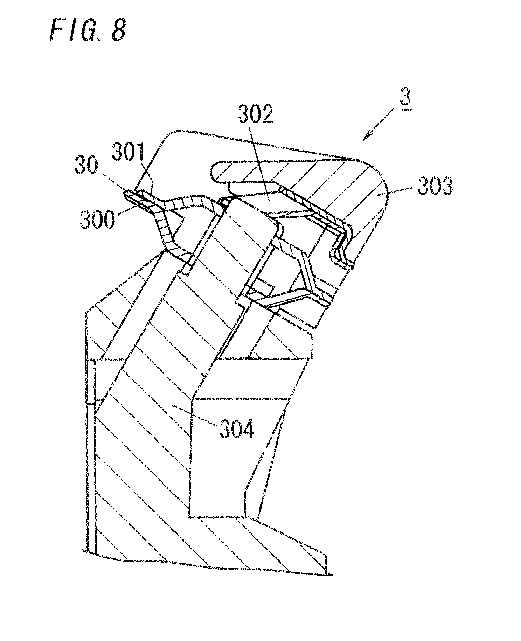

As shown in FIG. 5, the trimmer blade 30 includes a comb-shaped stationary blade 300 that is formed in a long line shape perpendicular to the light axis direction, and a comb-shaped movable blade 301 that is arranged along the stationary blade 300 and is formed in a line shape. As shown in FIG. 8, in the trimmer blade 30, the stationary blade 300 and the movable blade 301 are overlapped, and the movable blade 301 is arranged on the body surface 9 side. Moreover, the trimmer blade 30 has a spring 302 that biases the movable blade 301 elastically toward the stationary blade 300. Furthermore, the trimmer blade 30 has a trimmer cover 303 that covers this movable blade 301 from its tip end side and is provided over the entire length of the stationary blade 30 in a longitudinal direction. The spring 302, whose one end is fixed to the trimmer cover 303 and whose other end contacts the movable blade 301, thus biases the movable blade 301 toward the stationary blade 300.

The trimmer blade 30 includes a trimmer base 304 that supports and fixes the stationary blade 300, the movable blade 301, and the trimmer cover 303. As shown in FIG. 5, the trimmer base 304 is formed lengthwise along the height direction of the side face of the frame 12. The trimmer base 304 includes a support portion 305 that supports the lever 310 by being connected to the connection portions 311 that are provided protruding from the lever 310, and a driving lug 306 that is provided at approximately the center of the trimmer blade 30 in a longitudinal direction and is for causing the movable blade 301 to move back and forth along the stationary blade 300.

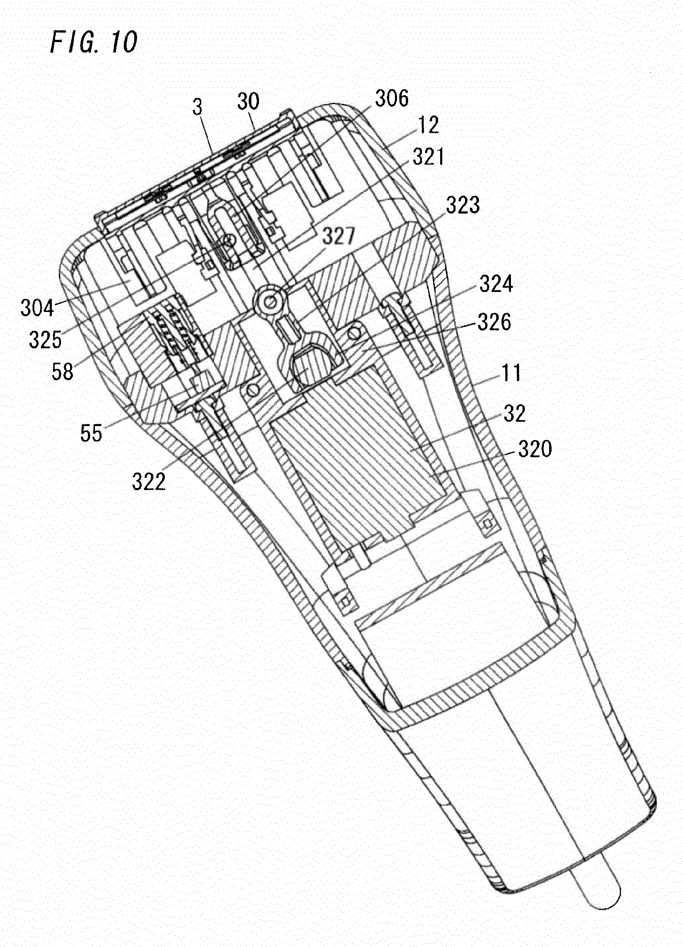

The support portion 305 according to the present embodiment has a fitting hole 307. Also, the driving lug 306 is configured to oscillate in a direction parallel to the longitudinal direction of the trimmer blade 30, and causes reciprocating movement of the movable blade 301 in a longitudinal direction by this oscillation. The driving lug 306 according to the present embodiment is configured by a fork-shaped lug, as shown in FIG. 10, which opens toward the base end side in a light axis direction (obliquely downward in an example shown in FIG. 10).

The trimmer blade 30 according to the present embodiment is configured so that it can be retracted in (slide) parallel to the light axis direction. The trimmer blade 30 moves (slides) following the movement (slide) of the operation means 31 that can move (slide) between a first end and a second end parallel to the light axis direction. The first end of the operation means 31 is located at an advancing direction side in the light axis direction, and the second end of the operation means 31 is located at a base end side in the light axis direction.

As shown in FIG. 5, the operation means 31 includes a lever 310 that is provided at the frame 12 to be movable (slidable) between the first end and the second end parallel to the light axis direction, and the lever 310 includes the connection portion 311 that are provided protruding from a back face of the lever 310 to the inside of the frame 12 through the through-hole 21.

The lever 310 has an approximately rectangular shape in a front view, and is provided with a protruding bar portion 312 to be caught by fingers at a front face thereof. The lever 310 is attached slidably to a lever mounting portion 20 of the frame 12.

The connection portions 311 are connected to the trimmer blade 30. Specifically, the connection portions 311 are inserted to the through-holes 21 provided at the lever mounting portion 20 of the frame 12 and are connected to the support portions 305 of the trimmer base 304. The connection portions 311 according to the present embodiment are configured by protruding portions that are provided protruding from a back face of the lever 310. The connection portions 311 according to the present embodiment are connected by fitting them into fitting holes 307 of the trimmer base 304 through the through-holes 21 of the frame 12.

When a user moves the lever 310 to the first end side (obliquely upward in FIG. 1), the connection portions 311 that are provided protruding from the lever 310 move upward, the trimmer base 304 moves upward following them, and the trimmer blade 30 protrudes from the beauty device (specifically the mounting part 15). When the trimmer blade 30 is protruded from the beauty device, if a user moves the lever 310 to the second end side thereof (obliquely downward in the example shown in FIG. 2), the connection portions 311 that are provided protruding from the lever 310 move downward, the trimmer base 304 moves downward following them, and the trimmer blade 30 is retracted into the beauty device (specifically mounting part 15). The beauty device is configured so that the tip of the trimmer blade 30 is substantially flush with the end face portion 22 of the frame 12, as shown in FIG. 2, or is set back further from the end face portion 22, when the trimmer blade 30 is retracted into the beauty device completely.

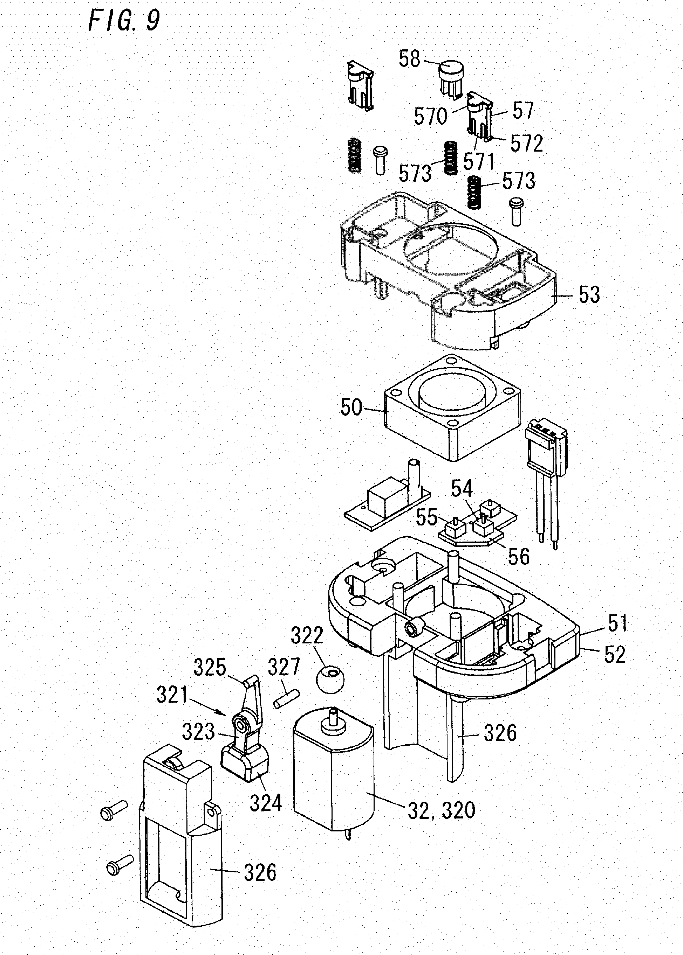

As shown in FIGS. 9 and 10, the driver 32 includes a motor 320 as a drive source, and an oscillation mechanical portion 321 that is provided between the rotation axis of the motor 320 and the driving lug 306 of the trimmer base 304, and transmits the drive force of the motor 320 to the driving lug 306. The driver 32 further includes a motor base portion 326 that accommodates the motor 320 and the oscillation mechanical portion 321 therein.

The oscillation mechanical portion 321 includes an eccentric cam 322 that is fixed to the rotation shaft of the motor 320, and an oscillation lever 323 whose approximate center is axis-supported by the motor base portion 326, and which is oscillated parallel to the longitudinal direction of the trimmer blade 30 by the eccentric cam 322. The oscillation lever 323 has a cam connection portion 324 that is connected to the eccentric cam 322, and an actuating portion 325 that is located at the opposite side of the cam connection portion 324. The cam connection portion 324 is opens to its front side (lower side in the example shown in FIG. 9) and to one of its lateral sides. As shown in FIG. 10, both end portions of the cam connection portion 324 in the same direction as the longitudinal direction of the trimmer blade 30 are in contact with the eccentric cam 322. And, in the direction perpendicular to the longitudinal direction (that is, in the direction parallel to the light axis direction), as shown in such as FIG. 2, there is a space between the cam connection portion 324 and the eccentric cam 322, so that the eccentric cam 322 does not come into contact with the cam connection portion 324 there.

The actuating portion 325 is connected to the driving lug 306 of the trimmer blade 30. The actuating portion 325 causes the movable blade 301 of the trimmer blade 30 to move back and force by oscillating around a central shaft 327. Note that there is a space between the actuating portion 325 and the crotch portion of the fork-like driving lug 306, as shown in FIG. 10, when the trimmer blade 30 is protruded from the beauty device. Therefore, when a user has retracted the trimmer blade 30 into the beauty device, the trimmer base 304 can be moved toward the base portion side of the main body 11 (moved downward), without being obstructed by the actuating portion 325.

The motor base portion 326 is fixed to a fan base portion 51 that is fixed inside the casing 10, and fixes the motor 320 inside the casing 10.

When the motor 320 rotates, the eccentric cam 322 rotates, and the eccentric cam 322 presses the cam connection portion 324 in a direction parallel to the longitudinal direction of the trimmer blade 30. Thereupon, the cam connection position 324 oscillates around the central shaft 327, and following this, the actuating portion 325 oscillates around the central shaft 327. The actuating portion 325 causes the driving lug 306 of the trimmer base 304 to oscillate, and causes the movable blade 301 of the trimmer blade 30 to move back and forth. Accordingly, the trimmer blade 30 causes the movable blade 301 to move back and forth along the longitudinal direction of the stationary blade 300, and cuts hair grown on a body surface 9.

The light-irradiating part 4 is arranged adjacently to the cutter 3 in this configuration.

The light-irradiating part 4 has the function of imparting a hair-growth-regeneration suppression effect on a body surface 9 by irradiating light on the body surface 9. The light-irradiating part 4 according to the present embodiment is located at a back side of the cutter 3 with respect to a movement direction of the beauty device main body 1, and light irradiation can be performed on the body surface 9 just after the hair thereon has been cut by the cutter 3.

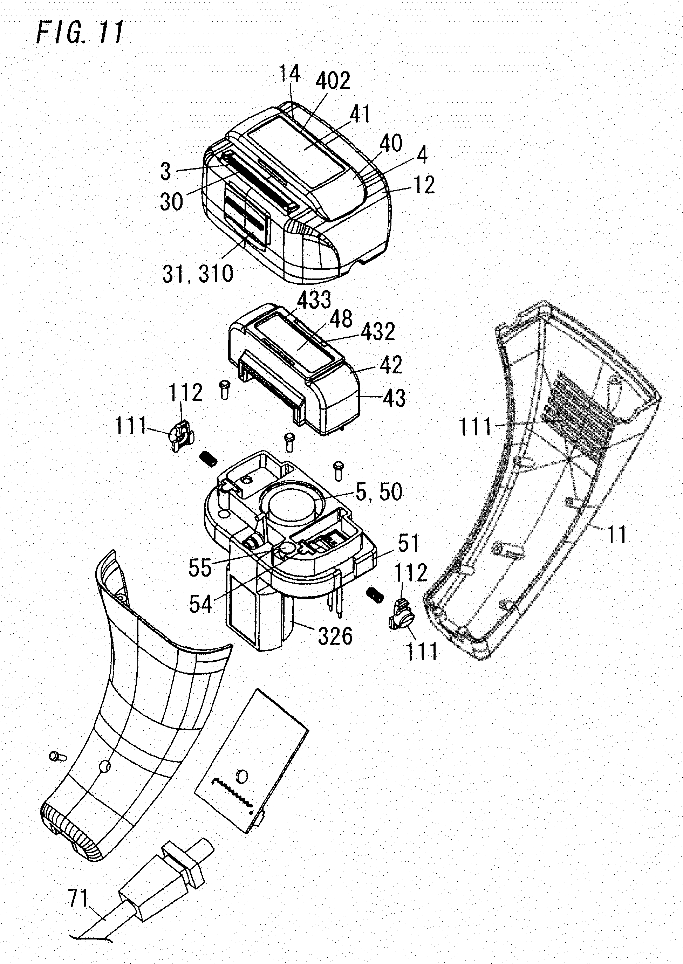

As shown in FIG. 11, the light-irradiating part 4 is configured by a light-shielding tube portion 40 that is movable in the light axis direction, and an irradiating unit 42 that is accommodated in the light-shielding tube portion 40 and has an emission member (emission portion) 44 therein.

The light-shielding tube portion 40 prevents the light from the emission member 44 from leaking outside by the end face (upper face) thereof being pressed to a body surface 9 and being brought in close contact with the body sulfate 9, when the emission member 44 emits light. The light-shielding tube portion 40 is mounted to the mounting part 14 of the frame 12 slidably in the light axis direction. The light-shielding tube portion 40 according to the present embodiment is biased in a direction protruding from the mounting part 14 (i.e. upward). The hght-shielding tube portion 40 causes the emission member 44 to emit light when it is located at the lowest position, in other words, it acts also as an emission switch (details will be described later). As shown in FIG. 5, the light-shielding tube portion 40 is formed in a rectangular tube shape that has an irradiation opening 402 at its upper side, and is open to its lower side. The end face (upper face) of the hght-shielding tube portion 40 is a body contact face 401.

In the light-shielding tube portion 40, a transparent member 41 is fitted into the irradiation opening 402, and the irradiation opening 402 is covered by the transparent member 41. The transparent member 41 according to the present embodiment is configured by a transparent plate member with a high transmission factor. The surface (upper face) of the transparent member 41 is set back from the body contact face 401 of the light-shielding tube portion 40. The transparent member 41 is positioned between the irradiating unit 42 and the body surface 9, and prevents the irradiating unit 42, which takes on a high temperature due to the emission from the emission member 44, from contacting the body surface 9. Furthermore, the transparent member 41 prevents hair scraps cut by the cutter 3 from adhering to a lens 48 of the irradiating unit 42.

At a side face of the light-shielding tube portion 40, a ventilation commmication opening 403 is provided penetrating therethrough. The ventilation communication opening 403 is an opening that ejects air sent from a blowing fan 50 serving as a cooling unit 5 to the outside. As shown in FIG. 4, the ventilation opening 432 is located upward of the end face portion 22 of the frame 12, when the light-shielding tube portion 40 is mounted to the frame 12 and the light-shielding tube portion 40 is in a natural state.

The light-shielding tube portion 40 has guide protrusions 404 that are provided at a plurality of positions and protrude sideward, which are to be fitted inside of guide grooves 181 formed at the guide faces 18 of the mounting part 14. The light-shielding tube portion 40 further has a plurality of engaging portions 405 to engage respectively with a plurality of (for example two) counter-engaging portions 19 provided at the mounting part 14.

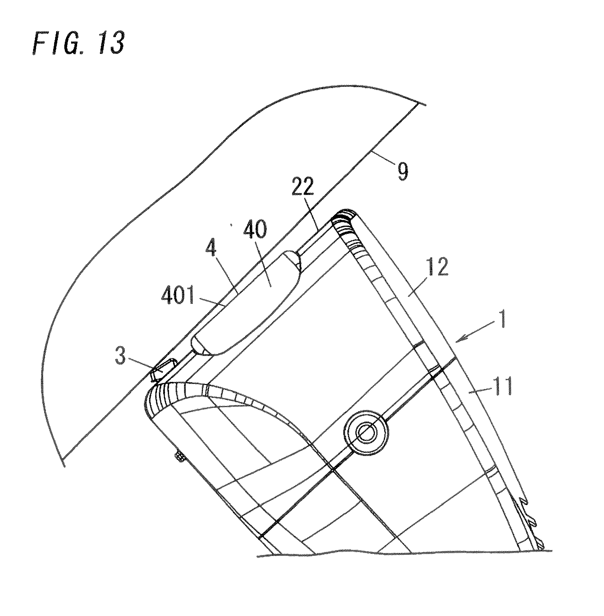

The light-shielding tube portion 40 (contact portion) is movable back and forth between a first end and a second end parallel to the light axis direction, and the first end of the light-shielding tube portion 40 is located at an advancing direction side in the light axis direction, and the second end of the light-shielding tube portion 40 is located at a base end side in the light axis direction. When the light-shielding tube portion 40 is located at the second end (lower end), the body contact face 401 (end face of the contact portion) is substantially flush with the front end of the trimmer blade 30 protruded (completely) from the beauty device (refer to FIG. 13).

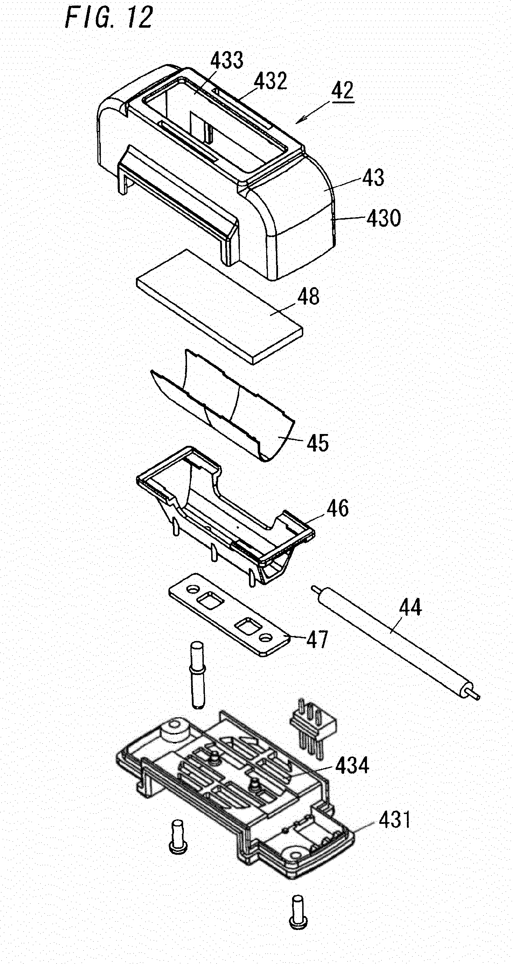

As shown in FIG. 12, the irradiating unit 42 has carious parts included inside a case portion 43, and constitutes one unit. The light-irradiating part 4 includes the case portion 43, which includes an emission member accommodating member 430 and a bottom face member 431, and the emission member 44, which is accommodated in the case portion 43 and serves as a light source. The light-irradiating part 4 further includes a reflector 45 that reflects and converges the light emitted from the emission member 44 toward the body surface 9, and a light source base portion 46 that holds the emission member 44 and the reflector 45. Further, the light-irradiating part 4 has an elastic member 47 that is inserted between the reflector 45 and the light source base portion 46, and elastically holds the emission member 44 and the reflector 45.

The emission member accommodating member 430 is configured by a rectangular tube shaped portion that has an irradiation window 433 at an end face (upper face), which passes light from the emission member 44, and opens to one direction (downward in the example shown in FIG. 12). This irradiation window 433 is formed in an elongated rectangular shape, and a transparent lens 48 is provided so as to cover the opening thereof. The lens 48 according to the present embodiment has a function of an optical filter that cuts unnecessary wavelengths such as ultraviolet light from the light irradiated from the emission member 44. The lens 48 is mounted to a frame portion of the light source base portion 46, and is installed in a state in which it contacts an inner face (lower face) of an outer edge of the irradiation window 433.

At an outer edge portion of the irradiation window 433, the emission member accommodating member 430 is provided with a plurality of ventilation openings 432 that penetrate the case portion 43. The ventilation openings 432 according to the present embodiment are slit-like openings provided along a longitudinal direction of the irradiation window 433, and eject air sent from the blowing fan 50 from inside the case portion 43 to inside the light-shielding tube portion 40. At an opening of the emission member accommodating member 430, the bottom face member 431 is installed.

This bottom face member 431 is attached so as to close the opening of the emission member accommodating member 430. The bottom face member 431 has a lattice-like communication hole 434 that introduces the air blown from the cooling unit 5 into the case portion 43. This communication hole 434 penetrates the case portion 43 so as to put the inside and outside in communication.

The emission member 44 is a light source of the light-irradiating part 4, and is configured by a linear and tube-shaped xenon flash lamp. The emission member 44 is arranged so that its longitudinal direction is approximately parallel to the reciprocating direction of the movable blade 301. The emission member 44 is connected electrically to the power supply unit 70, and when the light-shielding tube portion 40 is pushed (to the side of the bottom face member 431 (lower side)) and arrives at the second end thereof, an irradiation switch is turned on and a high voltage is applied to the emission member 44. When a high voltage is applied to the emission member 44, it irradiates light pulses with an extremely short emission time. Moreover, the emission member 44 continues to emit light pulses at a constant interval, when the light-shielding tube portion 40 is moved to the base end side (lower side) in the light axis direction and is held at the second end thereof.

The reflector 45 has an approximately U shaped cross-section, and is arranged so that its opening faces the irradiation window 433. The reflector 45 reflects and collects the light emitted from the emission member 44 with high efficiency, and guides the light toward the irradiation window 433. The emission member 44 is arranged at an inward portion of a flexed portion of the reflector 45.

As shown in FIG. 1, the irradiating unit 42 is mounted and fixed in the mounting part 14 in the frame 12. The irradiating unit 42 is arranged adjacent to the cutter 3, and a partition wall 16 is inserted between the cutter 3 and the irradiating unit 42. The irradiating unit 42 is installed so that a space 8 is formed between the end face thereof (upper face) and the rear face of the body contact face 401 of the light-shielding tube portion 40, as a movement space of the light-shielding tube portion 40 in the light axis direction. Whereas this irradiating unit 42 is fixed to the frame 12, the light-shielding tube portion 40 that covers the irradiating unit 42 from its base end side (upper side) is slide-movable in the light axis direction.

At the base end side (lower side) of the light-irradiating part 4 in this configuration, the cooling unit 5 is provided so that it neighbors the light-irradiating part 4.

The cooling unit 5 is a unit to cool the light-irradiating part 4, and is constituted by a blowing fan 50. The cooling unit 5 according to the present embodiment is fixed to the front end (upper end portion) of the main body 11, and is arranged adjacent to the light-irradiating part 4, when the frame 12 is mounted to the main body 11. As shown in FIG. 11, the cooling unit 5 includes a fan base portion 51 that is fixed to cover the front opening of the main body 11, and the blowing fan 50 that is fixed to the fan base portion 51.

As shown in FIG. 9, the fan base portion 51 includes a base portion main body 52 and a covering portion 53 that is attached from a front end side (upper side) of the base portion main body 52. The covering portion 53 is installed to the base portion main body 52 from the upper side in a state in which the blowing fan 50 is accommodated in it. Thus, the blowing fan 50 is fixed to the fan base portion 51.

When the blowing fan 50 is driven in a state in which the frame 12 is mounted to the main body 11, the blowing fan 50 introduces air from an air supply opening 110 that is provided at a side face of the main body 11. The air that is introduced from the air supply opening 110 ventilates from the communication hole 434 at the case portions 43 to the ventilation opening 432 through the blowing fan 50. The air that ventilates at this time ventilates while contacting the emission member 44 and the lens 48, which have taken on a high temperature, and performs heat exchange. The air that has received heat by the heat exchange ventilates into the light-shielding tube portion 40 through the ventilation opening 432, and is ejected outside through a ventilation communication opening 403 of the light-shielding tube portion 40.

Furthermore, at the base portion main body 52 of the fan base portion 51, as shown in FIG. 9, a switch 54 for the light-irradiating part that is to cause the emission member 44 of the light-irradiating part 4 to emit light, and a switch 55 for the cutter 3 that is to stop driving the movable blade 301 of the cutter 3 are provided. Both of these switches, namely the switch 54 for the light-irradiating part and the switch 55 for the cutter, are arranged on a circuit board 56, and are connected electrically to the power supply unit 70 through the circuit board 56 and the cable 71.

At the covering portion 53 of the fan base portion 51, a pair of pressing lugs 57 that press the light-shielding tube portion 40 to its end face side (upper side) are provided. These pressing lugs 57 each include a contact portion 570 that contacts the tip (lower end) of the pressing protrusion 406 of the light-shielding tube portion 40, a switch pressing portion 571 that presses the switch 54 for the light-irradiating part due to the contact portion 570 being pressed to the retracted-into side (lower side) of the light-shielding tube portion 40 into the beauty device, and a locking claw 572 that works as a stopper towards the upper side. The pressing lugs 57 are biased upward by coil springs 573.

When the hght-shielding tube portion 40 is retracted into the beauty device (moves downward), the pressing protrusion 406 of the light-shielding tube portion 40 presses down the pressing lug 57 against the biasing force. When the hght-shielding tube portion 40 is pressed further and reaches the second end, the switch pressing portion 571 of the pressing lug 57 turns on the switch 54 for the light-irradiating part. Accordingly a high voltage is applied to the emission member 44, and pulsed light is emitted from the emission member 44. Moreover, the light-shielding tube portion 40 is located at the first end in the forward side in the light advancing direction, since it is biased upward by the biasing force of the pressing lug 57, in a natural state in which the body surface 9 does not contact the body contact face 401.

Further, at the covering portion 53 of the fan base portion 51, a cutter pressing lug 58 is provided to press down the switch 55 for the cutter. This cutter pressing lug 58 presses down the switch 55 for the cutter due to being pressed down by a front end portion (lower end portion) of the trimmer base 304. The cutter pressing lug 58 is, similar to the pressing lug 57, biased upward by a coil spring 573.

When a user slides the lever 310 to its second end side in order to retract the trimmer blade 30 into the beauty device, the trimmer base 304 presses the cutter pressing lug 58 against the biasing force. When the lever 310 reaches the second end (lowest position) to retract the trimmer blade 30 into the beauty device, the cutter pressing lug 58 presses the switch 55 for the cutter, and stops the driver 32 for the cutter 3. In other words, the driver 32 stops to move the movable blade 301.

Thus, in the light-irradiating beauty device according to the present embodiment, a switch for light-irradiation and a switch for stopping the driver 32 for the cutter 3 are configured by different switches. Accordingly, it is configured so that the emission by the light-irradiating part 4 is possible, regardless of the position of the trimmer blade 30 relative to the beauty device (mounting part 15). Therefore, for example, even in a case where the trimmer blade 30 is retracted into the beauty device (mounting part 15 of the frame 12), the light-irradiating part 4 can perform light-irradiation on the body surface 9.

In the light-irradiating beauty device according to the present embodiment, the cutter 3 includes: the retractable trimmer blade 30 including the movable blade 301 and the stationary blade 300; and the operation means 31 configured to protrude the trimmer blade 30 from the light-irradiating beauty device or retract the trimmer blade 30 into the light-irradiating beauty device. Therefore, the user is capable of using only the light-irradiating part 4 by retracting the trimmer blade 30 into the beauty device when the cutter 3 is not used. As a result, it is possible to irradiate light without pressing the trimmer blade 30 on the body surface 9 without hair.

In the light-irradiating beauty device according to the present embodiment, when the trimmer blade 30 is retracted into the beauty device, the moving of the cutter 3 is stopped. Therefore, when stopping the use of the cutter 3, the user is capable of stopping the cutter 3 by only retracting the trimmer blade 30 into the beauty device by the operation means 31. Therefore, it is unnecessary to perform the action for stopping the movement of the cutter 3 before retracting the trimmer blade 30 into the beauty device by the operation means 31. As a result, it is possible to not only improve the usability but also prevent the stop operation of the cutter 3 from being forgotten.

In the light-irradiating beauty device according to the present embodiment, the light-irradiating part 4 is configured to being capable of irradiating light regardless of the position of the trimmer blade 30 relative to the beauty device (flame 12 or mounting part 15). Therefore, it is possible to perform the operation for protruding the trimmer blade 30 from the beauty device or retracting the trimmer blade 30 into the beauty device while irradiating light.

In the light-irradiating beauty device, the trimmer blade 30 is connected to the slidable lever 310 provided on the flame. Therefore, it is possible to protrude the trimmer blade 30 from the beauty device or retract the trimmer blade 30 into the beauty device by the slide of the lever 310, with simplified mechanism. It is possible to reduce costs by simplifying this mechanism.

In the light-irradiating beauty device, the end face of the light-shielding tube portion (contact portion) 40 on the base end side (second terminal) of the light axis 44a is substantially flush with the tip end of the trimmer blade 30 retracted into the beauty device. Therefore, it is possible to improve the touch to the skin in the wide area of the body surface 9 such as an arm or a leg. Furthermore, the user irradiates light easily while cutting hair and the usability improves.

Note that, in the light-irradiating part 4 according to the present embodiment, although a xenon flash lamp is used in the emission member 44, the light-irradiating part 4 of the present invention is not limited to this.

While the invention has been described with respect to some of the preferred embodiments, it will be imderstood that various changes and modification may be made by those skilled in the art without departing from the spirit and scope of the invention as defined in the following claims.

## Claims

1. A light-irradiating beauty device comprising:
a cutter (3); and
a light-irradiating part (4) that is placed beside the cutter (3), and is configured to irradiate light,
wherein the cutter (3) comprises:
a retractable trimmer blade (30) comprising a movable blade (301) and a stationary blade (300);
an operation means (31) configured to protrude the trimmer blade (30) from the light-irradiating beauty device or retract the trimmer blade (30) into the light-irradiating beauty device; and
a driver (32) configured to drive the movable blade (301) along the stationary blade (300),
wherein the driver (32) is configured to stop moving the movable blade (301) when the trimmer blade (30) has been retracted into the light-irradiating beauty device by the operation means (31).

2. The light-irradiating beauty device according to claim 1, wherein the light-irradiating part (4) is configured to irradiate light regardless of a position of the trimmer blade (30) relative to the light-irradiating beauty device.

3. The light-irradiating beauty device according to claim 1 or 2, further comprising a frame (12) to which the cutter (3) is attached,
wherein the operation means (31) comprises:
a connection portion connected to the trimmer blade (30) via a through-hole (21) of the flame (12); and
a slidable lever (310) provided on the flame (12),
wherein the trimmer blade (30) is configured to protrude from or retract into the light-irradiating beauty device depending on a slide of the lever (310).

4. The light-irradiating beauty device according to one of claims 1 to 3,
wherein the light-irradiating part (4) comprises:
an emission portion (44); and
a contact portion (40) which is free to move in a direction of a light axis (44a) extending from the emission portion (40),
wherein an end face of the contact portion on a base end side of the light axis (44a) is substantially flush with a tip end of the trimmer blade (30) protruded from the light-irradiating beauty device.
